# EUROPEAN PATENT SPECIFICATION

(11) **EP 1 471 937 B1**
(45) Date of publication and mention of the grant of the patent: **22.07.2009**
(21) Application number: 03704569.7
(22) Date of filing: 05.02.2003
(51) Int. Cl.: A61K 39/255, C12N 7/00, C12N 5/16, C07K 14/055, C12N 7/02, C12N 15/85, C12N 15/00

(54) **A CONTINUOUS CELL LINE FOR THE PRODUCTION OF VACCINES**
EINE KONTINUIERLICHE ZELLINIE ZUR HERSTELLUNG VON IMPFSTOFFEN
LIGNEE CELLULAIRE CONTINUE PERMETTANT DE PRODUIRE DES VACCINS

(30) Priority: 06.02.2002 EP 02075480
(43) Date of publication of application: 03.11.2004
(73) Proprietor: Lohmann Animal Health GmbH & Co. KG, 27454 Cuxhaven (DE)
(72) Inventor: OSTERRIEDER, Nikolaus, 87734 Benningen (DE); SCHUMACHER, Daniel, Ithaca, NY 14850 (US)
(74) Representative: Winckels, Johannes Hubertus F.
(86) International application number: PCT/EP2003/001213
(87) International publication number: WO 2003/066093

(56) References cited:
- EP-A- 0 748 867
- EP-A- 0 775 743
- EP-A- 0 884 382
- SCHUMACHER DANIEL ET AL: "Glycoproteins E and I of Marek's disease virus serotype 1 are essential for virus growth in cultured cells." JOURNAL OF VIROLOGY, vol. 75, no. 23, December 2001 (2001-12), pages 11307-11318, XP001080219 ISSN: 0022-538X
- ABUJOUB A A ET AL: "Development of a cell line system susceptible to infection with vaccine strains of MDV" ACTA VIROLOGICA, ACADEMIA PRAGUE, PRAGUE, CS, vol. 43, no. 2-3, April 1999 (1999-04), pages 186-191, XP002165582 ISSN: 0001-723X

## Description

The invention relates to the field of immunology and biotechnology. In particular it relates to the production of a cell line suited for the production of a vaccine against animal diseases, more in particular avian diseases, caused by herpesviruses, more in particular alphaherpesviruses. It also relates to the production of a cell line suited for the isolation of existing and newly emerging alphaherpesviruses, such as for example avirulent, virulent and very virulent (or hypervirulent) Marek's disease virus strains.

Marek's disease (MD) is a devastating disorder that affects chickens worldwide. It is caused by Marek's disease virus (MDV), an *alphaherpesvirus,* and characterized by T cell lymphomas, polyneuritis, general immunosuppression, and rarely atherosclerosis ⁵. Three MDV serotypes have been identified, out of which only serotype 1 (MDV-1) is pathogenic, whereas MDV serotype 2 (MDV-2) and serotype 3 (herpesvirus of turkeys, HVT) do not cause MD. MDV infection results in the establishment of latent infection which is common in *alphaherpesvirinae,* however, MDV has unique properties when compared to other members of this virus subfamily because it can cause tumors and integrate into the host cell genome ^{11,12}**.**

MD can be controlled by immunization, but despite vaccination more and more pathogenic MDV strains have evolved ⁴. These strains have been classified as so-called virulent (v), very virulent (vv) or very virulent plus (vv+) strains ^{28,35}. Vaccination using HVT was capable to protect chickens against infections with vMDV, but failed to protect against newly emerging vvMDV in the late 1970's ³⁸. Vaccines containing MDV-2 strains or combinations of HVT and MDV-2 were used successfully in the 1980's ^{6,34,37,39}, but within few years novel vv+ MDV-1 strains like 648A and 584A were isolated which can break bivalent vaccination and cause acute transient paralysis as well as a significant increase in mortality within the first two weeks after infection ^{15,37}. The appearance of vv+MDV has led to the introduction of the CVI988/Rispens vaccine in the U.S. which has been used in Europe since the 1970's ^{21,22,36,40}.

The isolation of new highly virulent strains from vaccinated flocks has not only been reported in the U.S. but also in Europe. Changes of clinical signs and cellular tropism have been reported for strain C12/130 ³. The mechanisms underlying the steady increase in MDV virulence and the appearance of a more acute neuronal form of MD remain enigmatic, but vaccine formulations and application forms may have an impact on these phenomena ³⁸. MDV exhibits high cell association in vitro and MD vaccines, except for some HVT formulations, represent living chicken cells infected with the agent which are kept in liquid nitrogen until application ³⁰.

The in vitro cultivation of MDV is enormously tedious, laborious and difficult to standardize. It is now realised that some glycoproteins are essential for virus growth in cultured cells, that is, virus depleted of gE or gI for example does not spread in culture at all (Shumaccher et al, J. Virol. 75: 11307-11318, 2001). An important cost factor in the production of vaccines for a prevention of MDV infection and MD in the domestic chicken is the use of cell cultures. To date the efficient growth of MDV is possible only on primary chicken or duck cells. Usually, the cell culture of choice for production of MDV vaccines is chicken embryo fibroblast cells (CEF)³⁸. These CEF are also used for isolation of MDV obtained from clinical cases in the form of peripheral blood cells (PBC) or after induction of lytic MDV replication from transformed tumor T cells. Alternatively, for isolation of virulent and not cell culture adapted virus strains or isolates, primary chicken kidney cells (CKC) or duck embryo fibroblasts (DEF) can be used. These cells proved superior to CEF for isolation of MDV from clinical samples. All known systems that support growth of MDV in culture rely on the production of primary cell cultures which have to be prepared continuously from embryonated eggs or even hatched birds in the case of CKC. The procedure of preparing primary cells not only is laborious but also causes high costs. This is reflected by the fact that approximately 30% of the cost of vaccine production is caused by the preparation of chicken embryo fibroblasts. Vaccine production is very dependent on a continuous and reliable supply of fertile eggs from specified pathogen free (SPF) flocks. SPF flocks are raised under special conditions and are regularly demonstrated to be free of avian pathogens. Any disruption in the supply of fertile SPF eggs would disrupt production of MDV vaccines.

Even though virulent and field MDV strains have been successfully propagated and isolated on chicken kidney (CKC) or duck embryo fibroblast cells, vaccine MDV or virulent strains, only efficiently grow on CEF cells after adaptation. Many attempts to propagate an MDV strain on continuous cell lines of avian or mammalian origin have failed. The failures were either caused by the loss of important properties of the virus after growth on heterologous cells or by the fact that abortive infections only could be demonstrated. In the last decade, however, the generation of cell lines constitutively infected with MDV have been reported. It was for example shown by Abujoub et al (Acta Virologica 43: 186-191, 1999 and in EP 0 775 743) that CHCC-OU2 cells, permanent derivatives of CEF cells, could be latently infected with virulent or avirulent MDV strains. However, long periods of MDV adaptation to the cell culture system used were necessary, in which changes in the genetic or antigenetic composition of the individual viruses could occur. With the CHCC-OU2 cell line, 4 weeks of culture were needed before MDV-specific plaques were visible. The resulting CEF cell lines OU2.1 and OU2.2 contained a latent form of virulent and tumor-inducing MDV as long as the cells were not confluent. The switch from latent to lytic infection was induced as soon as cell-to-cell contacts within the cultures were built. The cells are latently infected with only one strain and - upon confluency and by some mechanism no one really understands - lytic cycle replication is turned on. In conclusion,in this system one needs a special cell line for each virus one wants to propagate, one needs long times of adaptation and the cells are difficult to grow. Additionally the virulent Md11 strain grown on OU-OCL2 cells still was able to induce tumors in susceptible chicken lines. Recently, a continuous Vero cell line was tested for its susceptibility for MDV growth. It was reported that, after several rounds of blind passaging and 3 weeks of adaptation, very low titers of MDV serotype 1 (MDV-1) and serotype 3 (MDV-3) (i.e. low levels of infectious virus) could be produced. As outlined previously, long adaptation to cell culture systems are prone to result in unwanted genetic alterations which then can cause e.g. vaccine failures when vaccine strains have to be adapted to these cells. The poultry industry has always recognized the need for continuous cell lines that could be used for producing Marek's disease vaccines. Although many avian or mammalian cell lines have been developed (see for example EP 0 748 867 and EP 0 884 382) , until the present invention, no continuous cell line developed could be an effective substitute for chicken embryo fibroblast (CEF) cells in vaccine production. The maximum titer of MDV virus recoverable from these continuous cell lines could not match the titer of virus recovered from primary cell lines. In addition many hypervirulent and virulent MDV strains could not be propagated on previously developed continuous cell lines nor on primary cell lines.

The invention provides a method for producing a continuous cell line, essentially free of an mammalian or avian virus, capable of supporting the growth of a herpesvirus strain comprising infecting or transfecting a cell with a vector comprising a nucleic acid fragment derived of a herpesvirus, preferably an alphaherpesvirus, and culturing said infected or transfected cell or progeny thereof under conditions suitable for the expression of said nucleic acid and propagation of said cell or progeny thereof. Considering that the cell line is essentially free of virus it is a prerequisite that such a nucleic acid does not encode for a replicating herpes virus itself, to allow for later infection with a herpesvirus strain to be propagated. Therefore, said continuous cell line as provided here is not in itself a (latent) virus producer, but is used to cultivate or grow a herpes virus after inoculation therewith to sufficient titres to for example produce a vaccine. It is for example sufficient that said nucleic acid or fragment thereof of a herpesvirus comprises a nucleic acid encoding a structural protein or glycoprotein or part thereof. Examples of herpesviruses include Varicella Zoster virus, cytomegalovirus, Epstein-Barr virus, herpes simplex virus etc. Thus the present invention among others provides a continuous cell line that can support the growth of herpesviruses, including hypervirulent, virulent and avirulent MDV strains without need for adaptation of said strains for vaccine production. In a preferred embodiment the invention provides the use of a cell infected or transfected with a vector comprising a nucleic acid or fragment thereof of a glycoprotrein E (gE) gene or functional fragment thereof. In a preferred embodiment the disclosure provides a nucleic acid wherein said nucleic acid comprises a Marek's disease virus glycoprotein gE gene or functional fragment thereof or a glycoprotein gE gene equivalent. Preferably said glycoprotein gE gene is derived from an alphaherpes virus. As used herein "a functional fragment thereof' refers to a gene/nucleic acid fragment whose expressed product retains the property of supporting/sustaining virus growth, preferably a herpes virus growth in said continuous cell line. A glycoprotein gE gene "equivalent" as used herein is any like gene (i.e. functional equivalent) for example a glycoprotein gE gene obtained from another herpesvirus, such as an alphaherpesvirus whose expressed product has the property of supporting/sustaining virus growth in said continuous cell line. In another preferred embodiment the disclosure provides a nucleic acid wherein said nucleic acid comprises a Marek's disease virus glycoprotein gL gene or functional fragment thereof or a glycoprotein gL gene equivalent. Preferably said glycoprotein gL gene is derived from an alphaherpes virus. As used herein "a functional fragment thereof' refers to a gene/nucleic acid fragment whose expressed product retains the property of supporting/sustaining virus growth, preferably a herpes virus growth in said continuous cell line. A glycoprotein gL gene "equivalent" as used herein is any like gene (i.e. functional equivalent) for example a glycoprotein gL gene obtained from another herpesvirus, such as an alphaherpesvirus whose expressed product has the property of supporting/sustaining virus growth in said continuous cell line. In another preferred embodiment the disclosure provides a nucleic acid wherein said nucleic acid comprises a Marek's disease virus glycoprotein gI gene or functional fragment thereof or a glycoprotein gI gene equivalent. Preferably said glycoprotein gI gene is derived from an alphaherpes virus. As used herein "a functional fragment thereof" refers to a gene/nucleic acid fragment whose expressed product retains the property of supporting/sustaining virus growth, preferably a herpes virus growth in said continuous cell line. A glycoprotein gI gene "equivalent" as used herein is any like gene (i.e. functional equivalent) for example a glycoprotein gI gene obtained from another herpesvirus, such as an alphaherpesvirus whose expressed product has the property of supporting/sustaining virus growth in said continuous cell line. In a preferred embodiment, the invention provides a method for producing a continuous cell line capable of supporting the growth of a Marek's disease virus strain comprising infecting or transfecting a cell with a vector comprising a nucleic acid or fragment thereof of a Marek's disease virus strain and culturing said infected or transfected cell or progeny thereof under conditions suitable for the expression of said nucleic acid and propagation of said cell or progeny thereof. It is a requisite that such a nucleic acid does not encode for replicating Mark's disease virus itself, to allow for later infection with a herpesvirus strain to be propagated. A continuous cell line as used herein is an established/sustainable cell line, which can be grown without restrictions and can undergo at least 10 passages. Again, it is desired that said continuous cell line, albeit provided with a nucleic acid fragment derived from a herpesvirus, be free of mammalian and avian viruses, as, incidentally, was found to be not the case for the quail cell line QT-35 which turned out to be latently infected with an MDV. By use of the term "cell or progeny thereof " it is understood that said cell can be cultured and expanded from a single cell to produce a cell line using several commercially available culture media, under known conditions suitable for propagating avian cells. By "infection" or "transfection" is meant the DNA transfer of virus or fragment(s) thereof into avian or mammalian cells. Methods for gene transfer into cells are known to those skilled in the art. For example the method of transfection may comprise known techniques used to transfect DNA into cells, such as calcium phosphate precipitation, polybrene, DEAE-dextran, LIPOFECTIN, electroporation, or protoplast fusion.

A vector as used herein comprises any gene delivery vehicle which is capable of delivering nucleic acid to said cell, such as for example a plasmid vector (pcDNA3, Invitrogen). Nucleic acid as used herein refers to a nucleic acid sequence of a herpesvirus, preferably an alphaherpesvirus, and even more preferred a Marek's disease virus such as for example a gene equivalent to the glycoprotein E (gE) of alphaherpesviruses or a fragment thereof. The preferred Marek's disease virus strain is selected from a Marek's disease virus oncogenic serotype-1 (MDV-1) and/or nononcogenic serotype-2 (MDV-2) and/or nononcogenic serotype-3, turkey herpesvirus (HVT). Such a nucleic acid may be expressed constitutively or transiently under the control of the cell's own regulatory elements or heterologous regulatory elements (i.e. constitutively active regulatory elements or inducible regulatory elements). In a preferred embodiment said nucleic acid its expressed product has the functional property of supporting/sustaining the growth of a virus, preferably avian viruses, [e.g. Marek's disease virus, Varicella Zoster virus, Newcaster Disease Virus (NDV), Infectious Bursal Disease Virus (IBDV), Infectious Bronchitis Virus (IBV), Chicken Anemia Virus (CAV), Infectious Laryngotracheitis Virus (ILV), Avian Leukosis Virus (ALV), Reticuloendotheliosis Virus (REV) and Avian Influenza Virus (AIV) etc.] in said continuous cell line.

Various culturing conditions for said cell line, including media formulations with regard to specific nutrients, oxygen, tension, carbon dioxide and reduced serum levels, can be selected and optimised by one of skill in the art. Preferably said infected or transfected cell according to the invention comprises a vertebrate cell. Even more preferred said infected or transfected cell comprises an avian cell. For example said cell may be derived from muscle tissue, i.e. a muscle myoblast cell. Any muscle myoblast cell may be utilized in accordance with the present invention for example said muscle myoblast cell may be obtained from human, primate, chicken, quail, turkey, goose, pigeon, pheasant, bobwhite muscle tissue. Muscle cell lines can be cultured and maintained using known vertebrate cell culture techniques. In a preferred embodiment said myoblast cell is obtained from quail such as for example a QM cell. A preferred QM cell is a QM7 cell deposited as ATCC CRL-1632.

In a preferred embodiment the disclosure provides a nucleic acid wherein said nucleic acid comprises a Marek's disease virus glycoprotein gE gene or functional fragment thereof or a glycoprotein gE gene equivalent. Preferably said glycoprotein gE gene is derived from a Marek's disease virus serotype 1 and/or serotype 2 and/or serotype 3. As used herein "a functional fragment thereof" refers to a gene/nucleic acid fragment whose expressed product retains the property of supporting/sustaining virus growth, preferably a Marek's disease virus growth in said continuous cell line. A glycoprotein gE gene "equivalent" as used herein is any like gene (i.e. functional equivalent) for example a glycoprotein gE gene obtained from another herpesvirus, such as an alphaherpesvirus whose expressed product has the property of supporting/sustaining or enhancing virus growth in said continuous cell line. In another preferred embodiment said Marek's disease virus comprises an avirulent Marek's disease virus strain such as for example Rispens CVI988. The application encompasses all attenuated Marek's disease virus/vaccine strains that are capable of replication in said continuous cell line. The invention provides a method for producing a continuous cell line capable of supporting the growth of a Marek's disease virus strain comprising infecting or transfecting a cell with a vector comprising a nucleic acid or fragment thereof of a Marek's disease virus strain and culturing said infected or transfected cell or progeny thereof under conditions suitable for the expression of said nucleic acid and propagation of said cell or progeny thereof, wherein said continuous cell line is capable of supporting the growth of a Marek's disease virus strain without adaptation of said virus. "Without adaption"as used herein means that said continuous (i.e. permanent / sustainable) cell line according to the invention is capable of supporting the growth and productive infection of a Marek's disease virus at high titers (i.e. at a level comparable or even superior to that of a primary cell line, e.g. chicken embryo fibroblast cells) after 1 or 2 passages.

In yet another embodiment the invention provides a cell or progeny thereof obtainable by a method according to the invention. Furthermore the invention provides a cell preparation obtained from a cell or progeny thereof according to the invention. Preferably said cell or progeny thereof or cell lysate is infected or transfected with a Marek's disease virus serotype 1 attenuated strain. The invention provides use of a cell infected or transfected with a vector comprising a nucleic acid or fragment thereof of a Marek's disease virus strain according to the invention and/or a preparation derived therefrom for the preparation of a vaccine capable of inducing some measure of protection against a disease (i.e. to produce immunity) in a vertebrate, preferably an avian species. The present invention relates also to a method for the preparation of Marek's disease virus in quantities suitable for vaccine purposes. The invention provides a method to prepare a vaccine capable of inducing an immune response against disease in a vertebrate, preferably an avian species, comprising culturing a cell according to the invention and harvesting cell culture components therefrom. It is also understood that said Marek's disease virus strain can be maintained as a non-lytic or a lytic infection in said cell line (i.e. SOgE), and said cell line according to the invention is able to infect vertebrates, preferably avians in vivo, in order to produce immune protection against Marek's Disease

Furthermore the application provides a method for producing both monovalent and multivalent vaccines for protecting vertebrates, preferably avian species against infection by disease causing agents, i.e. in addition to Marek's disease virus. It is understood that said cell line according to the invention (i.e. SOgE) can be infected or transfected with a vector such as a Marek's disease virus vector comprising a Marek's disease virus genome or parts thereof including genes encoding one or more heterologous proteins or polypeptides (i.e. antigenic peptides of disease causing agents) which are useful against vertebrate disease causing agents other than a Marek's disease virus. Examples of disease causing agents which may be useful for producing vaccines against include Newcastle Disease Virus (NDV), Infectious Bursal Disease Virus (IBDV), Infectious Bronchitis Virus (IBV), Chicken Anemia Virus (CAV), Infectious Laryngotracheitis Virus (ILV), Avian Leukosis Virus (ALV), Turkey Astrovirus Reticuloendotheliosis Virus (RV) and Avian Influenza Virus (AIV). It is understood that virtually any heterologous gene sequence can be constructed in said vector comprising said nucleic acid for use in the preparation of vaccines i.e. mutivalent vaccines. It is preferable that such a heterologous gene sequence will encompass a gene product that can serve to boost or activate the host's cellular and/or humoral immune response, or a gene product that encodes an epitope that induces a protective immune response to any variety of pathogens (preferably vertebrate pathogens, even more preferred avian pathogens), or antigens that bind neutralizing antibodies. For example genes or gene fragments encoding antigenic peptides of one or more of the three serotypes of Marek's disease virus. Such antigenic peptides may be used in a multivalent vaccine to be administered to a vertebrate so as to confer a complete protective effect against infection with a Marek's disease virus.

The invention further provides a vaccine obtainable by a method according to the invention. Such vaccines can be prepared by methods well known to those skilled in the art of preparation of vaccines. Vaccines may take the form of suspensions of a cell line infected or transfected with said vector comprising a nucleic acid or fragment thereof of a Marek's disease virus strain, infected with and supporting the productive replication of a vaccine strain/attenuated strain of a Marek's disease virus, preferably serotype 1 and/or serotype 2 and/or serotype 3 (i.e. singly, i.e. monovalent vaccines) or in combinations thereof (i.e. multivalent vaccines)), and/or other viruses, like herpesviruses. Additionally a vaccine according to the invention may be made from cell free virus suspensions made from sonicated cells, e.g. SogE cells, and/or cell lysates or components of lysates infected with and supporting the productive infection of a vaccine strain/attenuated strain of a Marek's disease virus preferably serotype 1 and/or serotype 2 and/or serotype 3 (i.e. singly or in combination thereof) and/or other viruses, like herpesviruses. It is understood that a vector according to the disclosure may be engineered using recombinant techniques within the skill of those in the art to encode heterologous proteins which are known to boost a vertebrate immune response (i.e. capable of stimulating class 1or 2 major histocompatibility complex molecule presentation), thus enhancing the overall protective effect of an administered vaccine.

The invention provides the use of a cell infected or transfected with a vector comprising a nucleic acid or fragment thereof of a Marek's disease virus strain for the generation and/or maintenance and/or isolation of a herpesvirus, preferably an alphaherpesvirus. Examples of herpesviruses include Varicella Zoster virus, cytomegalovirus, Epstein-Barr virus, herpes simplex virus etc. In a preferred embodiment the invention provides the use of a cell infected or transfected with a vector comprising a nucleic acid or fragment thereof of a Marek's disease virus strain for the generation and/or maintenance and/or isolation of a hypervirulent and/or virulent and/or avirulent Marek's disease virus strain. Such a cell can be used as a suitable substrate for the propagation of hypervirulent strains, such as the EU1, 648A and 584A strain, and/or virulent strains such as the RB1B strain and/or avirulent strains such as 584Ap80C and CVI988. The disclosure encompasses all Marek's disease virus strains selected from the group consisting of serotype 1, serotype 2, serotype 3. Serotype 1 includes all pathogenic strains and their attenuated derivatives. Serotype 2 consists of naturally avirulent chicken viruses, while serotype 3, also known as Herpesvirus of Turkeys (HVT), includes avirulent turkey viruses that are capable of replication in chickens. It is understood that said Marek's disease virus strain may comprise a natural or a genetically modified strain.

The present invention encompasses a method to generate and/or isolate and/or maintain a very virulent (vv), a very virulent plus (vv+), a hypervirulent and/or virulent and/or avirulent Marek's disease virus strain comprising infecting a cell capable of supporting the growth of a Marek's disease virus strain with a herpesvirus and culturing said cell under conditions suitable for the propagation of said cell and the generation, maintenance and isolation of said herpesvirus. The disclosure furthermore provides a herpesvirus obtainable by a method according to the invention.

In a preferred embodiment the invention provides a method to generate and/or isolate and/or maintain very virulent (vv), a very virulent plus (vv+), a hypervirulent and/or virulent and/or avirulent Marek's disease virus strain comprising infecting a cell capable of supporting the growth of a Marek's disease virus strain with a very virulent (vv), a very virulent plus (vv+), hypervirulent and/or virulent and/or avirulent Marek's disease virus strain and culturing said cell under conditions suitable for the propagation of said cell and the generation, maintenance and isolation of said very virulent (vv), a very virulent plus (vv+), hypervirulent and/or virulent and/or avirulent Marek's disease virus strain. Suitable conditions for culturing vertebrate/avian cells are known in the art. The disclosure furthermore provides a very virulent (vv), a very virulent plus (vv+), hypervirulent and/or virulent and/or avirulent Marek's disease virus strain obtainable by a method according to the invention.

Additionally it is preferred that said cell capable of supporting the growth of a Marek's disease virus strain is cultured in the presence of geneticin (G-418), derivative or analog thereof, or any other drug that is nontoxic in the presence of a neomycin resistance gene and can be used for selection. A derivative of G-418 as used herein is a substance derived from G-418 with like properties. An analog of G-418 as used herein is a substance possessing a chemical structure and chemical properties similar to that of G-418 (Gibco, Life Technologies).

The invention further provides the use of a cell capable of supporting the growth of a Marek's disease virus strain according to the invention for the production of a diagnostic antigen of a herpesvirus, preferably an alphaherpesvirus and even more preferred a Marek's disease virus. It is understood that virtually any heterologous gene sequence can be constructed in said vector comprising said nucleic acid for use in the preparation of a diagnostic antigen. For example genes or gene fragments encoding non common antigenic peptides of one or more of the three serotypes of Marek's disease virus, or gene or gene fragments encoding specific antigenic peptides of other herpesviruses. Such specific viral antigens may be used to diagnose a specific viral infection in a bird and are also useful for vaccine production. Furthermore the invention provides a method for the production of a diagnostic antigen of a Marek's disease virus comprising providing a cell according to the invention infected with and capable of supporting the growth of a Marek's disease virus strain and isolating components therefrom

The invention further provides a vaccine according to the invention for the preparation of a medicament for the prevention and/or treatment of a vertebrate disease. Preferably said vertebrate disease is an avian disease, even more preferred a Marek's disease virus associated disease. Suitable basis for a medicament are known in the art. The invention includes both prophylactic and therapeutic vaccines. The diclosure further provides a method for protecting a vertebrate against a disease comprising administering to said vertebrate a vaccine according to the invention with a pharmaceutically acceptable carrier to a suitable recipient. The disclosure further provides a method for immunizing a vertebrate (e.g. animal, human, poultry) against infectious herpesvirus comprising administering to a vertebrate an effective immunizing dose of a vaccine according to the present invention. Either a live recombinant viral vaccine or an inactivated recombinant viral vaccine can be formulated. Production of live virus vaccine formulations can be accomplished using conventional methods involving propagation of the virus in a cell line according to the invention followed by administration as a medicament to a vertebrate. Vaccines may be administered to a vertebrate by any of the methods well known to those skilled in the art, for example by intramuscular, subcutaneous, intraabdominal, intravenous, or in-ovo injection. It is preferable to introduce the virus vaccine formulation via the natural route of infection of the pathogen for which the vaccine is designed. Alternatively such a vaccine may be administered oculo-nasally or orally. In order to prepare inactivated vaccines, the virus may be grown in cell line according to the invention , inactivated by for example formaldehyde or beta- propiolactone. Inactivated viruses may be formulated with a suitable adjuvant in order to enhance the immunological response. Such adjuvants may include, but are not limited to, mineral gels, e.g., aluminum hydroxide; surface active substances such as lysolecithin, pluronic polyols, polyanions; peptides; oil emulsions; and potentially useful adjuvants such as BCG and Corynebacterium parvum. Pharmaceutically acceptable carriers are well known in the art and include, but are not limited to, saline, buffered saline, dextrose, water, glycerol, sterile isotonic aqueous buffer, and combinations thereof. One example of such an acceptable carrier is a physiologically balanced culture medium containing one or more stabilizing agents such as stabilized, hydrolyzed proteins, lactose, dimethylsulfoxide etc. The carrier is more preferably sterile. A suitable recipient is a vertebrate, preferably an avian species.

### Example 1

**Animals**. White Lohmann selected leghorns (LSL) (Lohmann, Cuxhaven, Germany) were used and wing-banded on the day of hatch. Birds were kept in cages, and received food and water *ad libitum.*

**Cells and viruses.** Chicken embryo fibroblast cells (CEF) were prepared as described previously (Schumacher et al., 2000). Quail muscle QM7 cells (ATCC cell number CRL-1632) were used for preparation of the permanent cell line. The MDV strains used were CVI988 (MarekVac forte^{®}, Lohmann, Cuxhaven, Germany), 584Ap80C ³⁷, RB1B (kindly provided by Dr. T.F. Davison, Compton, UK), and EU1. Strain 584Ap80C or its US2-negative derivative BAC20 virus reconstituted from an infectious BAC clone of MDV (Schumacher et al., 2000) were propagated on CEF²⁹. MDV strain EU1 is a virus isolated in 1992 from a flock in Italy which had been immunized with an HVT vaccine. EU1 was shown to be free of chicken infectious anemia virus, avian leukosis and reticuloendotheliosis viruses, and infectious bursal disease virus by PCR, and could not be propagated on CEF. For preparation of virus, strain EU1 was injected intramuscularly (i.m.) into 10 chickens. At day 10 after infection, peripheral blood (PBMC) and spleen mononuclear cells were harvested from infected birds by Histopaque^{®} density centrifugation (Sigma, Munich, Germany) and stored in liquid nitrogen.

**Plasmid**. The glycoprotein E open reading frame, the US8 homologous gene 17, 33 of MDV vaccine strain Rispens CVI988 was amplified by polymerase chain reaction (PCR) using 100 pmol each of the 5'-primer 5'-CATAAGCATGCGAGTCAGCGTCATAATGTG-3'. and the 3'-primer 5'-CAAGGGCCCATCAGTGGTATAAATCTAAGC-3'. The resulting 1.4 kbp fragment was cleaved with restriction endonucleases BamHI and EcoRI and cloned in the same sites of vector pcDNA3 (Invitrogen) leading to recombinant vector pcMgE ³⁰. **PCR analysis.** One PCR assay targeted the gE open reading frame (see above), the other PCR assay performed on infected cells targeted the gB gene of MDV (Lee et al., 2000). The PCR assay ²⁵ targeting the MDV gB gene ^{17,33} was done with 100 pmol each of forward (5'-GCATATCAGCCTGTTCTATC-3') and reverse primer (5'-AACCAATGGTCGGCTATAAC-3'). In both protocols, the respective primers were mixed with DNA, and 35 cycles (95°C 30 s, 50°C 30 s, 72°C 30 s) were run. The specificity of PCR products was confirmed by Southern blotting using Digoxigenin-labeled gB or gE sequences as a probe ²⁹.

**Indirect immunofluorescence analysis.** For indirect immunofluorescence analyses (IIF), cells were grown on 6-well plates (Greiner) or on glass coverslips. Cells were fixed with 90% acetone at various times after infection, IIF was done exactly as described, and samples were analyzed by conventional fluorescence microscopy (29,30). The antibodies used were anti-gB monoclonal antibody (mab) 2K11; kindly provided by Dr. J.-F. Vautherot, INRA, Nouzilly, France) or a convalescent serum from a chicken infected with MDV-1 (anti-MDVI) ²⁹

### ELISA procedure to determine MDV-1 antibodies

MDV-1-specific antibodies in plasma were determined by enzyme-linked immunosorbant assays (ELISA). The antigen consisted of a lysate of 5 X 10⁷ BAC20-infected CEF harvested 5 days after infection by freeze-thawing. The lysate (5 ml in PBS) was sonicated for 60 s at 60 W, and cell debris was removed by centrifugation (10,000 g, 10 min, 4°C). A lysate of 5 X 10¹ uninfected CEF was used as the negative control. ELISA plates (Nunc) were coated with 100 µl of lysates (final concentration: 5 µl lysate/ml) for 16 hours at 4°C. Plasma samples were diluted in log₂-steps starting with a 1: 100 dilution and added to plates for 60 min at 25°C after blocking using 2% skim milk. After thorough washing with PBS, 100 µl anti-chicken IgG peroxidase conjugate (Sigma) was added for 30 min at 25°C. After final washing with PBS, 100 µl TMB substrate solution (Sigma) was added for 10 min before the reaction was stopped with 2 M H₂SO₄. Absorbances at 450 nm (A₄₅₀) were read (TecanSPECTRA, Crailsheim, Germany), and end-point antibody titers were determined as previously²⁰.

### Example 2: Generation of a cell line constitutively expressing glycoprotein E of MDV vaccine strain CVI988.

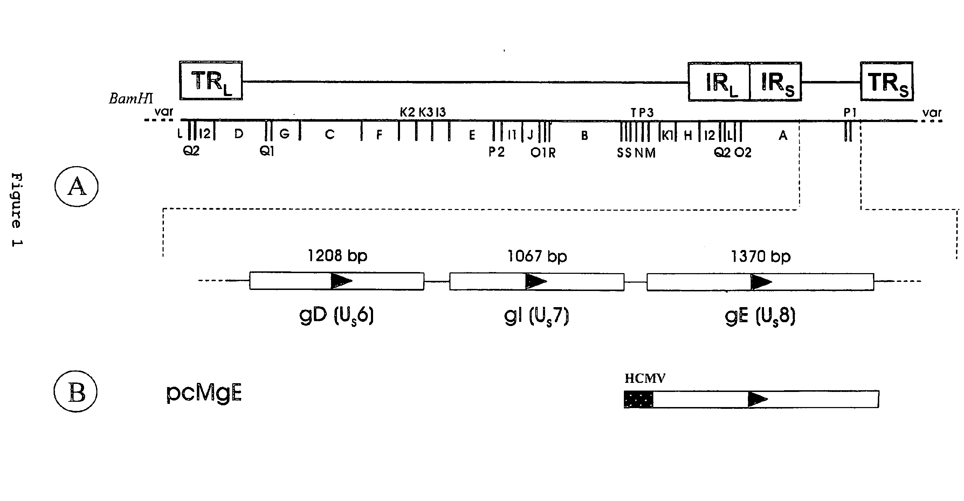

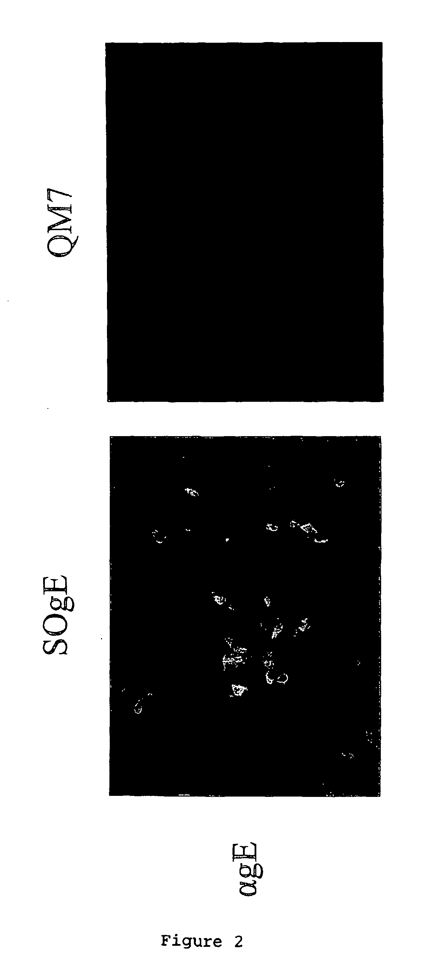

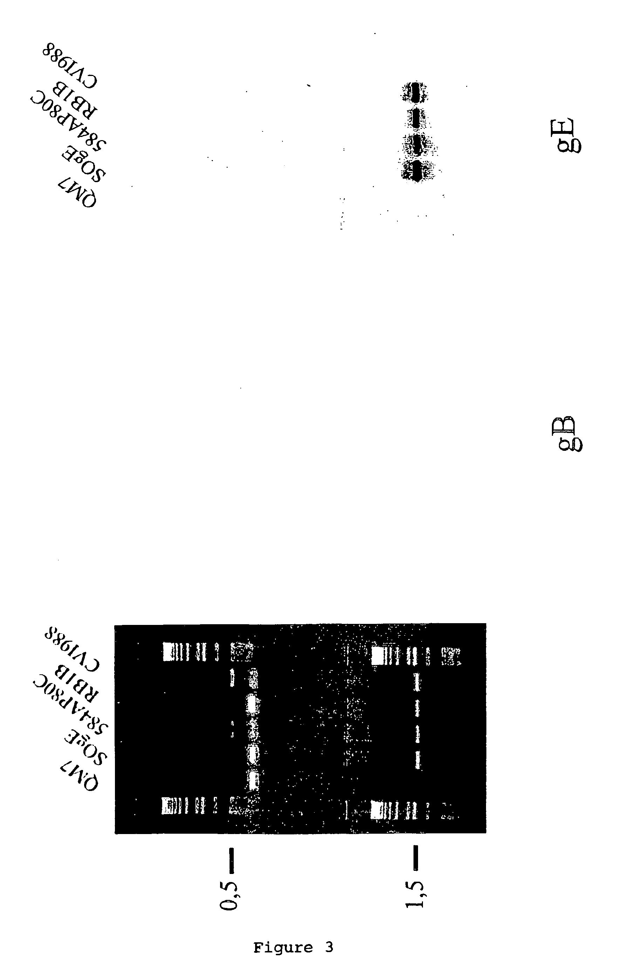

QM7 cells were transfected by the calcium phosphate precipitation method as described earlier ²⁹. QM7 cells were grown on 6-well plates until approximately 70% confluency and transfected with 10 µg of recombinant plasmid pcMgE ³⁰. Transfected cells were overlaid with DMEM medium containing 5% of FCS and 1000 µg per ml of G-418 (Gibco-BRL). After 2 weeks of cell culture in the presence of the antibiotic, single cell clones were picked into 96-well plates. After cell clones had grown to confluency in 96-well plates, they were split 1:2 and IIF using the convalescent MDVI serum was performed on the cell clones. A cell clone in which virtually every cell expressed MDV gE was identified (Figure 2), and termed SOgE. SOgE cells were expanded and checked for gE expression in weekly intervals. In the presence of G-418, gE expression proved stable for more than 15 weeks. In the absence of the drug, gE expression in SOgE cells was detected for up to 10 weeks. Presence of MDV gE DNA in SOgE but not in QM7 cells was confirmed by PCR analysis of DNA prepared from 1 X 10⁷ cells each (Figure 3).

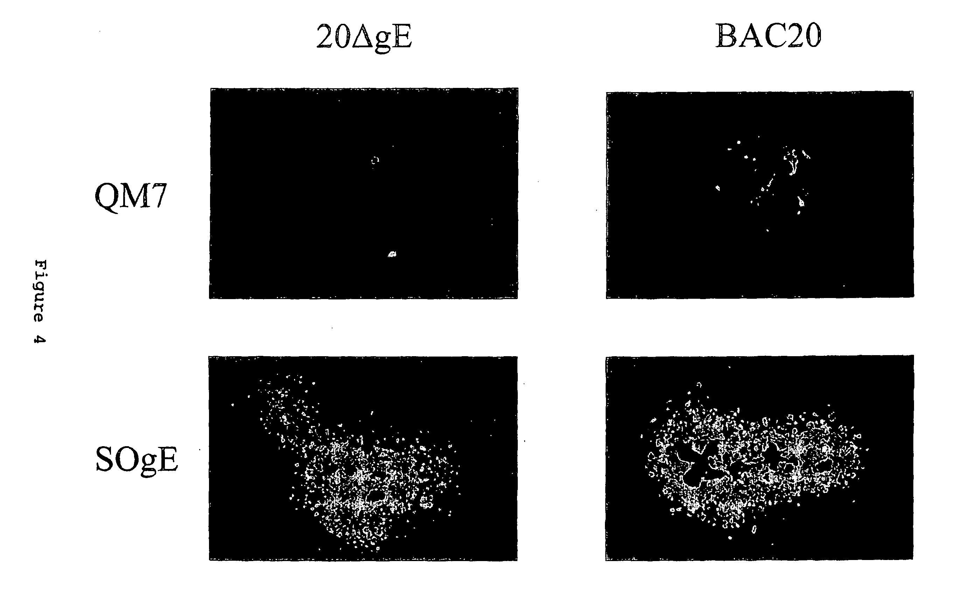

**Example 3: SOgE cells express functional MDV gE.** The question of expression of functional MDV gE by the generated cell line was addressed by the analysis of growth of a gE-negative MDV on SOgE cells. Glycoprotein E has been shown to be essential for MDV growth *in vitro* ³⁰. Therefore, 20ΔgE DNA encoding gE-negative BAC20 virus (Schumacher et al., 2000) was transfected into SOgE or QM7 cells and plaque formation was monitored. Whereas 20ΔgE plaques were readily observed on SOgE cells after IIF using anti-gB mab 2K11, only single infected cells could be visualized with the mab on parental QM7 cells (Fig. 4). These results confirmed that functional gE was produced by SOgE cells which was able to trans-complement the essential MDV-1 gE in a gE-deficient virus.

### Example 4 :SOgE cells support growth of several MDV-1 strains.

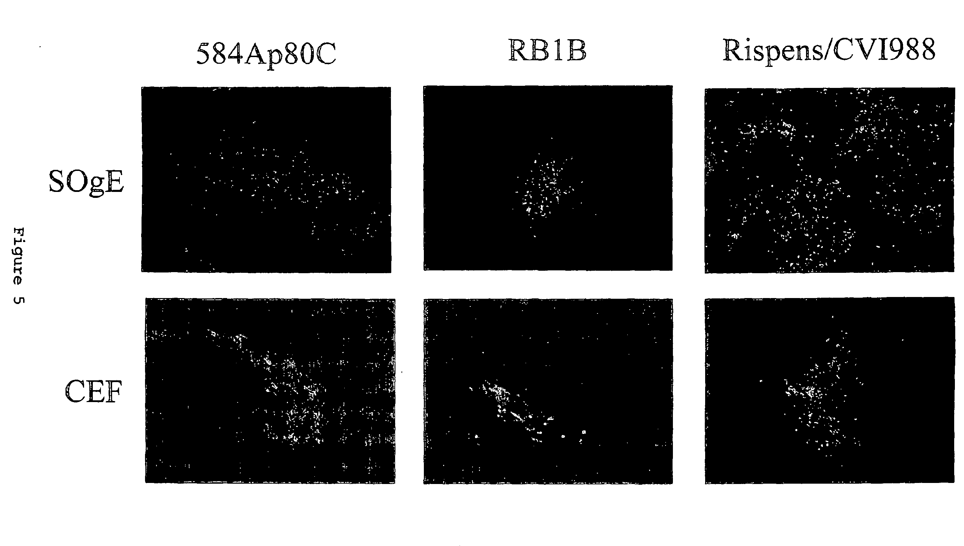

The size of 20ΔgE plaques on SOgE cells appeared much larger than that of BAC20 virus on QM7 cells or that of a gB-negative virus mutant on gB expressing QM7 cells ²⁹. This unexpected observation was addressed in further experiments. Plaque sizes of the avirulent MDV-1 strains 584Ap80C and CVI988 as well as the virulent RB1B and hypervirulent strain were assessed on CEF, QM7 and SOgE cells. This was done by co-seeding of infected CEF or PBMC (EU1) with the respective cells. It could be demonstrated that co-seeding of SOgE cells with CEF cells infected with 584Ap80C, CVI988, or RB1B at low multiplicity of infection (MOI = 0.0001; i.e. 100 PFU per 1 X 10⁶ cells) led to plaque formation (Fig. 5). The plaque sizes were comparable to those on CEF cells (Fig. 5). In addition, it was possible to directly reconstitute infectious MDV-1 from viral DNA or *Escherichia coli*-derived cloned viral DNA (BAC20), ²⁹ after transfection of SOgE cells (Fig. 5). Thus, direct adaptation of MDV to SOgE cells without lengthy cell culture passaging is possible, which is a major advantage for both vaccine production and isolation or generation of virulent and hypervirulent MDV-1 for animal experiments.
The above described experiments had suggested that SOgE cells represent a permanent cell substrate for efficient propagation of both avirulent (vaccine) MDV strains and virulent, very virulent and hypervirulent MDV-1 strains. Because especially production of MD vaccines could be facilitated using a permanent cell line allowing propagation of MDV vaccine strains, CVI988 DNA was transfected into CEF, QM7, or SOgE cells. At 6 days after transfection when virus plaques were visible, cells were harvested and 1 X 10³ infected cells were co-seeded with 1 X 10⁷ uninfected cells of the matching cell type. At 5 days after infection of this large number of cells, infected cells were trypsinized and titrated in tenfold dilutions on freshly prepared CEF, QM7 or SOgE cells. At 4 days after titration, cells were fixed and numbers of virus plaques were determined after IIF staining using the 2K11 anti-gB mab antibody. It could be shown that mean CVI988 titers on SOgE cells reached 1.8 X 10⁶ PFU whereas titers of only 3.2 X 10³ were obtained on QM7 cells. The titers on SOgE cells were virtually identical to those on primary CEF cells. From the results of the plaque size determinations and the titration experiments we concluded that propagation of virulent and avirulent vaccine MDV strains on SOgE cells, which constitutively express MDV strain CVI988 gE, was as effective as or even superior to propagation on primary CEF cells.

### Example 5 : SOgE cells do not cause tumors in 1-day old chickens.

The fact that SOgE cells were derived from a chemically induced quail tumor raised the remote possibility that it may cause tumors in chickens after systemic application of whole cell preparations. To address this important issue, a total of 18 chickens was inoculated with either SOgE cells (12 chickens) or parental QM7 cells (6 chickens). Each individual chicken received 1 X 10⁶ cells by the intramuscular and 1 X 10⁶ cells by the intraabdominal route (Table 1). The fate of the inoculated chickens was followed for a total of 12 weeks, when post-mortem examination was performed. None of the chickens exhibited any clinical signs during the course of the experiment. Additionally, all chickens appeared in a good nutrition state and without any sign of tumor formation at the post-mortem examination. From these results we concluded that recombinant MDV gE-expressing SOgE cells as well as parental QM7 cells do not cause tumors in chickens, even when approximately 100- to 1000-fold more cells compared to a vaccine dose are administered. Therefore, we considered SOgE cells as safe for the production of MDV or combined vaccines.

### Example 6:

### Example 6: Protection of chickens against Marek's Disease using SOgE produced vaccine

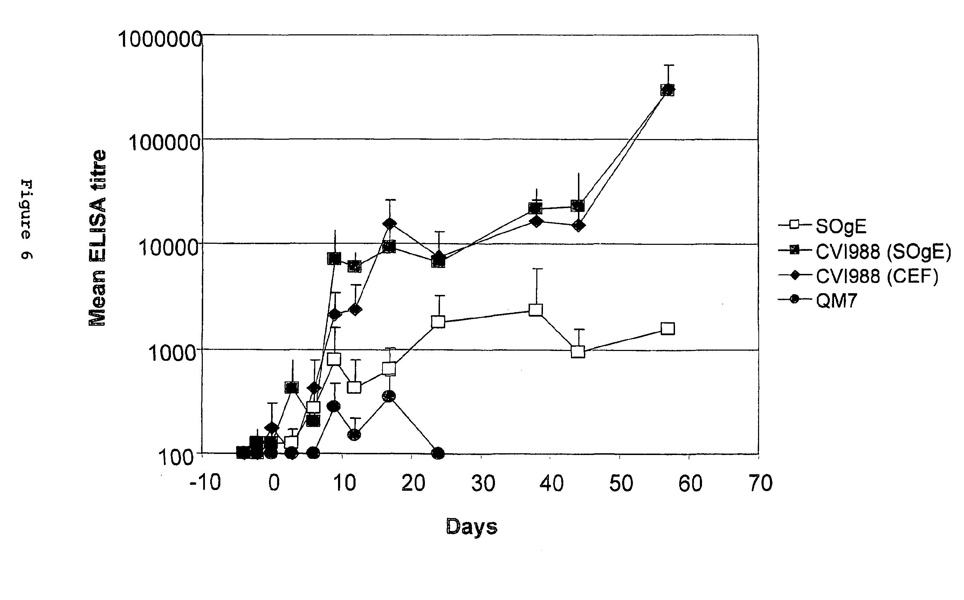

To compare the protective capacity of the vaccine strain Rispens CVI988 propagated on SOgE cells with that produced conventionally on CEF cells, animal experiments were performed. One-day-old chickens received 1 X 10⁶ SOgE cells (Group 1, Table 2), 1 X 10 parental QM7 cells (Group 4, Table 1) or 1 X 10³ plaque-forming units of CVI988 produced on SOgE (Group 2, Table 2) or CEF cells (Group 3, Table 2). To avoid cross-contamination, vaccine virus was produced after transfection of CVI988 DNA Isolated from CEF into the SOgE or CEF cells. Transfected cells were co-seeded with fresh uninfected cells and vaccine virus was harvested on Day 5 p.i. when complete cytopathic effect had developed. Immunised birds were challenge-infected with hypervirulent MDV-1 strain EU1 on Day 12 after immunization. In the mock-immunised animals (QM7 cells, group 4), chickens suffered from MD as early as 7 days after infection and two birds died on Day 9 and 10, respectively. By Day 37 p.i. all birds had died as a consequence of the infection (Table 2). In the SOgE-immunised animals, 4 chickens died as a consequence of the infection, but 3 birds survived until termination of the experiment on Day 70 after infection (Table 2). However, the 3 surviving birds exhibited MD as evidenced by gross pathology examination and the detection of tumours of inner organs (Table 2). In stark contrast, chickens immunised with CVI988, which was produced either on SOgE or on CEF cells, did not die from MD until the termination of the experiment (Table 2). In one bird each of Group 2 and 3, however, signs of MD were identified by gross pathology (Table 2).
Serological anti-MDV-1 responses in immunised and challenged birds were followed by performing ELISA testings. It could be demonstrated that protected birds exhibited ELISA antibody that were gradually rising with time after challenge infection, whereas chickens inoculated with SOgE or QM7 cells did not develop high antibody titers. Rather, antibody titers fell at later times after infection or remained virtually constant (Fig. 6). From the results it was concluded that the MD CVI988 vaccine which was produced on permanent SOgE cells provided as good a protection as that produced conventionally on CEF cells. In addition, protection against MD was coincident with gradually rising anti-MDV-1 antibodies, which may indicate that a permanent boost of the chicken immune systems by either lytically replicating vaccine virus or low level replication of EU1, which may be controlled by the presence of vaccine virus, is a prerequisite for protection against tumourigenic MD.

### References

**1.** Adler, H., M. Messerle, M. Wagner, and U. H. Koszinowski. 2000. Cloning and mutagenesis of the murine gammaherpesvirus 68 genome as an infectious bacterial artificial chromosome. J. Virol. 74:6964-6974.
2. Adler, H. E. and A. J. DaMassa. 1979. Toxicity of endotoxin to chicks. Avian Dis. 23:174-178.
3. Barrow, A. and K. Venugopal. 1999. Molecular characteristics of very virulent European MDV isolates. Acta Virol. 43:90-93.
4. Benton, W. J. and M. S. Cover. 1957. The increased incidence of visceral lymphomatosis in broiler and replacement birds. Avian Dis. 1:320-327.
5. Calnek, B. W. 2001. Pathogenesis of Marek's disease virus infection. Curr. Top. Microbiol. Immunol. 255:25-55.
6. Calnek, B. W., K. A. Schat, M. C. Peckham, and J. Fabricant. 1983. Field trials with a bivalent vaccine (HVT and SB-1) against Marek's disease. Avian Dis. 27:844-849.
7. Cui, Z., A. Quin, L. F. Lee, P. Wu, and H. J. Kung. 1999. Construction and characterization of a H19 epitope point mutant of MDV CVI988/Rispens strain: Acta Virol. 43 :169-173.
8. Cui, Z. Z., D. Yan, and L. F. Lee. 1990. Marek's disease virus gene clones encoding virus-specific phosphorylated polypeptides and serological characterization of fusion proteins. Virus Genes 3:309-322.
9. Davis, H. L. and M. J. McCluskie. 1999. DNA vaccines for viral diseases. Microbes. Infect. 1:7-21.
10. Davis, H. L., M. L. Michel, M. Mancini, M. Schleef, and R. G. Whalen. 1994. Direct gene transfer in skeletal muscle: plasmid DNA-based immunization against the hepatitis B virus surface antigen. Vaccine 12:1503-1509.
11. Delecluse, H. J. and W. Hammerschmidt. 1993. Status of Marek's disease virus in established lymphoma cell lines: herpesvirus integration is common. J. Virol. 67:82-92.
**12.** Delecluse, H. J., S. Schuller, and W. Hammerschmidt. 1993. Latent Marek's disease virus can be activated from its chromosomally integrated state in herpesvirus-transformed lymphoma cells. EMBO J. 12:3277-3286.
13. Fynan, E. F., R. G. Webster, D. H. Fuller, J. R. Haynes, J. C. Santoro, and H. L. Robinson. 1993. DNA vaccines: protective immunizations by parenteral, mucosal, and gene- gun inoculations. Proc. Natl. Acad. Sci. U. S. A 90:11478-11482.
14. Fynan, E. F., R. G. Webster, D. H. Fuller, J. R. Haynes, J. C. Santoro, and H. L. Robinson. 1995. DNA vaccines: a novel approach to immunization. Int. J. Immunopharmacol. 17:79-83.
15. Gimeno, I. M., R. L. Witter, and W. M. Reed. 1999. Four distinct neurologic syndromes in Marek's disease: effect of viral strain and pathotype. Avian Dis. 43:721-737.
16. Krishnan, B. R. 2000. Current status of DNA vaccines in veterinary medicine. Adv. Drug Deliv. Rev. 43:3-11.
17. Lee, L. F., P. Wu, D. Sui, D. Ren, J. Kamil, H. J. Kung, and R. L. Witter. 2000. The complete unique long sequence and the overall genomic organization of the GA strain of Marek's disease virus. Proc. Natl. Acad. Sci. U. S. A 97:6091-6096.
18. Messerle, M., I. Crnkovic, W. Hammerschmidt, H. Ziegler, and U. H. Koszinowski. 1997. Cloning and mutagenesis of a herpesvirus genome as an infectious bacterial artificial chromosome. Proc. Natl. Acad. Sci. U. S. A 94:14759-14763.
19. Morgan, R. W., J. L. Cantello, and C. H. McDermott. 1990. Transfection of chicken embryo fibroblasts with Marek's disease virus DNA. Avian Dis. 34:345-351.
20. Osterrieder, N., R. Wagner, C. Brandmuller, P. Schmidt, H. Wolf, and O. R. Kaaden. 1995. Protection against EHV-1 challenge infection in the murine model after vaccination with various formulations of recombinant glycoprotein gp14 (gB). Virology 208:500-510.
21. Rispens, B. H., H. van Vloten, N. Mastenbroek, H. J. Maas, and K. A. Schat. 1972. Control of Marek's disease in the Netherlands. I. Isolation of an avirulent Marek's disease virus (strain CVI 988) and its use in laboratory vaccination trials. Avian Dis. 16:108-125.
22. Rispens, B. H., H. van Vloten, N. Mastenbroek, J. L. Maas, and K. A. Schat. 1972. Control of Marek's disease in the Netherlands. II. Field trials on vaccination with an avirulent strain (CVI 988) of Marek's disease virus. Avian Dis. 16:126-138.
23. Robinson, H. L. and C. A. Torres. 1997. DNA vaccines. Semin. Immunol. 9:271-283.
24. Roy, K., H. Q. Mao, S. K. Huang, and K. W. Leong. 1999. Oral gene delivery with chitosan--DNA nanoparticles generates immunologic protection in a murine model of peanut allergy. Nat. Med. 5:387-391.
25. Saiki, R. K., T. L. Bugawan, G. T. Horn, K. B. Mullis, and H. A. Erlich. 1986. Analysis of enzymatically amplified beta-globin and HLA-DQ alpha DNA with allele-specific oligonucleotide probes. Nature 324:163-166.
26. Sambrook, J. and D.F.Fritsch and T.Maniatis. 1989. Molecular cloning: a laboratory manual. Cold Spring Harbor, N.Y..
27. SAS Institute Inc. 1984. SAS/STAT User's Guide, Version 6. SAS Institute Inc., Cary NC.
28. Schat, K. A., B. W. Calnek, and J. Fabricant. 1982. Characterisation of two highly oncogenic strains of Marek's disease virus. Avian Pathol. 11:593-605.
29. Schumacher, D., B. K. Tischer, W. Fuchs, and N. Osterrieder. 2000. Reconstitution of Marek's disease virus serotype 1 (MDV-1) from DNA cloned as a bacterial artificial chromosome and characterization of a glycoprotein B-negative MDV-1 mutant. J. Virol. 74:11088-11098.
30. Sharma, J. M. 1971. In vitro cell association of Marek's disease herpesvirus. Am. J. Vet. Res. 32:291-301.
31. Stokes, M. E., C. S. Davis, and G. G. Koch. 2000. Categorical Data Analysis Using the SAS System. SAS Institute Inc., Cary NC.
32. Suter, M., A. M. Lew, P. Grob, G. J. Adema, M. Ackermann, K. Shortman, and C. Fraefel. 1999. BAC-VAC, a novel generation of (DNA) vaccines: A bacterial artificial chromosome (BAC) containing a replication-competent, packaging- defective virus genome induces protective immunity against herpes simplex virus 1. Proc. Natl. Acad. Sci. U. S. A 96:12697-12702.
33. Tulman, E. R., C. L. Afonso, Z. Lu, L. Zsak, D. L. Rock, and G. F. Kutish. 2000. The genome of a very virulent Marek's disease virus. J. Virol. 74:7980-7988.
34. Witter, R. L. 1982. Protection by attenuated and polyvalent vaccines against highly virulent strains of Marek's disease virus. Avian Pathol. 11:49-62.
35. Witter, R. L. 1983. Characteristics of Marek's disease viruses isolated from vaccinated commercial chicken flocks: association of viral pathotype with lymphoma frequency. Avian Dis. 27:113-132.
36. Witter, R. L. 1992. Safety and comparative efficacy of the CVI988/Rispens vaccine strain, p. 315-319. World's Poultry Science Assoc., Amsterdam.
37. Witter, R. L. 1997. Increased virulence of Marek's disease virus field isolates. Avian Dis. 41:149-163.
38. Witter, R. L. 2001. Protective efficacy of Marek's disease vaccines. Curr. Top. Microbiol. Immunol. 255:57-90.
39. Witter, R. L. and L. F. Lee. 1984. Polyvalent Marek's disease vaccines: safety, efficacy and protective synergism in chicken with maternal antibodies. Avian Pathol. 13:75-92.
40. Witter, R. L., L. F. Lee, and A. M. Fadly. 1995. Characteristics of CVI988/Rispens and R2/23, two prototype vaccine strains of serotype 1 Marek's disease virus. Avian Dis. 39:269-284.
41. Yokoyama, H., R. C. Peralta, S. Sendo, Y. Ikemori, and Y. Kodama. 1993. Detection of passage and absorption of chicken egg yolk immunoglobulins in the gastrointestinal tract of pigs by use of enzyme-linked immunosorbent assay and fluorescent antibody testing. Am. J. Vet. Res. 54:867-872.

**Table 1: Testing of tumour formation in chickens after application of QM7 and SOgE cells**

| | | **Number of Animals with Tumors** | | | | | | | | | | | | |
|---|---|---|---|---|---|---|---|---|---|---|---|---|---|---|
| | | ***Weeks post inoculation*** | | | | | | | | | | | | |
| **Group** | **Animals (n)** | **0** | **1** | **2** | **3** | **4** | **5** | **6** | **7** | **8** | **9** | **10** | **11** | **Total** |
| SOgE | 6 | | | | | | | | | | | | **0** | 0/6 |
| QM7 | 6 | | | | | | | | | | | | **0** | 0/6 |

**Table 2: Marek's Disease in chickens immunised with vaccine strain CVI988 produced on SOgE cells and challenged with hypervirulent strain EU1**

| | | **Number of Animals with Marek's Disease** | | | | | | | | | | | |
|---|---|---|---|---|---|---|---|---|---|---|---|---|---|
| | | ***Weeks after infection*** | | | | | | | | | | | |
| **Group** | **Animals (n)** | **0** | **1** | **2** | **3** | **4** | **5** | **6** | **7** | **8** | **9** | **10** | **Total** |
| 1: SOgE | 7 | | | | | **1*** | **1** | | **2** | | | **3** | 7/7 |
| 2: CVI988-SOgE | 8 | | | | | | | | | | | **1** | 1/8 |
| 3: CVI988-CEF | 8 | | | | | | | | | | | **1** | 1/8 |
| 4: QM7 | 6 | | | **2** | | **2** | **2** | | | | | | 6/6 |

| | | | | | | | | | | | | | |
|---|---|---|---|---|---|---|---|---|---|---|---|---|---|
| *Numbers of animals given in weeks 0 to 9 died as a consequence of EU1 infection, numbers of chickens given in week 10 represent animals exhibiting MD at *post mortem* examination. | | | | | | | | | | | | | |

### Figure legends.

**Figure 1****:**
   Schematic representation of the MDV-1 genome and location of the gE open reading frame in the unique short region (Us). Shown is the organization of the approximately 180 kbp MDV-1 genome and the *Bam*HI restriction map. The gE open reading frame was amplified by PCR and cloned into pcDNA3 vector (Invitrogen) under the control of the human cytomegalovirus immediate early promoter/enhancer. Scales are given in kbp or bp.
**Figure 2****:**
   Detection of gE expression by SOgE cells by IIF using a rabbit polyclonal gE specific antiserum³⁰. Whereas reactivity of SOgE cells with the antibody was readily detected, no reactivity of the gE-specific serum with QM7 cells was observed. The individual views are 1,000 X 650 µm in size.
**Figure 3****:**
   Polymerase chain reaction of SOgE cells. SOgE cells were left uninfected or were infected with MDV-1 strains 584Ap80C, CVI988 or RB1B. Whereas gE-specific sequences were detected in all infected and uninfected SOgE cells, a gB-specific amplification product was obtained in infected cells only. In QM7 cells neither gB nor gE was detectable. The sizes of the amplification products are given. Specificity of the amplified products was verified using Digoxigenin-labeled gB or gE sequences as probes. Detection of DNA-DNA hybrids was done using CSPD^{™} (Roche Biochemicals) and enhanced chemoluminiscence according to the manufacturer's instructions.
**Figure 4****:**
   Growth of gE-negative and BAC20 virus on gE-expressing SOgE cells or QM7 cells. After transfection of 20ΔgE or BAC20 DNA (Schumacher et al., 2000,2001) into the respective cells, 20ΔgE plaque formation was visible from day 3 after transfection in SOgE but not in QM7 cells. In QM7 cells, only single infected cells but no plaque formation was observed in case of 20ΔgE virus. BAC20 virus caused very small plaques in QM7 cells but large plaques on recombinant SOgE cells. The individual views are 1,000 X 650 µm in size (lower panels) or 300 X 200 µm in size (upper panels).
**Figure 5****:**
   Plaques of MDV-1 strains 584Ap80C, RB1B and CVI988 on CEF and SOgE cells. Plaque formation induced by the vaccine strains 584Ap80C, CVI988 and the virulent MDV-1 strain RB1B were readily observed after plating on SOgE cells. Virus plaque formation on CEF cells was also observed in case of 584Ap80C, CVI988 and RB1B. The plaques shown were fixed at Day 4 after infection of 1 X 10⁶ cells with 100 PFU of the indicated viruses. The individual views are 1,500 X 1,000 µm in size.
**Figure 6****:**
   ELISA titers of chickens immunised with CVI988 produced on recombinant SOgE cells or on CEF. Day -12 is the Day of immunization, on Day 0 animals were challenged with hypervirulent MDV-1 strain EU1. Titers of MDV-1-specific antibodies in plasma. All birds of each group were bled on the indicated days and plasma samples of two birds were pooled. Plasma was diluted in log₂ steps starting at a 1:100 dilution. Titers are expressed as the dilution in which the A₄₅₀ₙₘ after reaction with MDV-infected cell lysates exceeded that with uninfected cell lysates by 3 standard deviations. Symbols for individual groups are explained.

## Claims

1. A method for producing a continuous cell line capable of supporting the growth of a cell associated alphaherpesvirus comprising infecting or transfecting a cell with a recombinant nucleic acid of a herpesvirus, and culturing said infected or transfected cell or progeny thereof under conditions suitable for the expression of said nucleic acid and propagation of said cell or progeny thereof, wherein said cell or progeny thereof is free of a mammalian or avian virus, and wherein said nucleic acid comprises a herpesvirus glycoprotein gE gene or functional fragment thereof.

2. A method according to claim 1 wherein said infected or transfected cell is a vertebrate cell.

3. A method according to claim 1 or claim 2 wherein said infected or transfected cell is an avian cell

4. A method according to anyone of claims 1-3 wherein said infected or transfected cell is a muscle myoblast cell.

5. A method according to claim 4 wherein said muscle myoblast cell is a QM7 cell.

6. A method according to claim 5 wherein said QM7 cell is derived from a cell deposited as ATCC CRL-1632.

7. A method according to anyone of claims 1-6 wherein said herpesvirus is a Marek's disease virus, preferably of serotype 1, 2 or 3.

8. A method according to claim 7 wherein said Marek's disease virus is an avirulent Marek's disease virus.

9. A method according to claim 8 wherein said avirulent strain is Rispens CVI988.

10. A method according to anyone of claims 1-9 wherein said continuous cell line is capable of supporting the growth of a Marek's disease virus strain without adaptation of said virus.

11. A cell or progeny thereof, free of a mammalian or avian virus, of a continuous cell line capable of supporting the growth of a cell-associated alphaherpesvirus comprising a recombinant nucleic acid of a herpesvirus, wherein said nucleic acid comprises a herpesvirus glycoprotein gE gene or functional fragment thereof.

12. A cell preparation obtained from a cell or progeny thereof according to claim 11.

13. Use of a cell according to claim 11 and/or a preparation according to claim 12 for the preparation of a vaccine capable of inducing protection against disease in a vertebrate.

14. Use according to claim 13 wherein said vertebrate is avian.

15. Use of a cell according to claim 11 for the generation and/or maintenance and/or isolation of a hypervirulent and/or very virulent plus, and/or very virulent strain, and/or virulent and/or avirulent Marek's disease virus strain.

16. Use according to claim 15 further comprising culturing said cell with said virus in the presence of geneticin.

17. Use according to claim 15 or claim 16 wherein said Marek's disease virus strain is a Marek's disease virus serotype 1.

18. Use according to anyone of claims 15-17 wherein said Marek's disease virus strain is EU1 or RB1B or 584Ap80C or CVI988.

19. Use according to anyone of claims 15-18 wherein said Marek's disease virus strain is a natural or genetically modified strain.

20. Use of a cell according to claim 11 for the production of a diagnostic antigen of a Marek's disease virus.

21. A method to generate and/or isolate and/or maintain a Marek's disease virus strain comprising infecting a cell according to claim 11 with said strain and culturing said cell under conditions suitable for the propagation of said cell and the generation, maintenance and isolation Marek's disease virus.

22. A method according to claim 21 comprising culturing said cell with said virus in the presence of geneticin.

23. A method according to claim 21 or claim 22 wherein said Marek's disease virus strain is a Marek's disease virus serotype 1, serotype 2, or serotype 3.

24. A method according to anyone of claims 21-23, wherein said Marek's disease virus strain is EU1 or RB1B or 584Ap80C or CVI988.

25. A method according to anyone of claims 21-24 wherein said Marek's disease virus strain is a vaccine strain.

26. A method for the production of a diagnostic antigen of a Marek's disease virus comprising providing a cell according to claim 11 and isolating said antigen therefrom.

27. A method to prepare a vaccine capable of inducing protection against disease in a vertebrate, comprising culturing a cell according to claim 11 and harvesting cells or virus-containing components there from.

28. A method according to claim 27 wherein said vertebrate is avian.

29. A vaccine capable of inducing protection against disease in a vertebrate, wherein the vaccine comprises harvested cell components from a cultured cell according to claim 11.

30. A vaccine according to claim 29 wherein said vertebrate is avian.

31. Use of a vaccine according to claim 29 in the preparation of a medicament for the prevention and/or treatment of a herpesvirus induced disease.

32. Use according to claim 31 wherein said disease is a Marek's disease virus associated disease.

33. Use according to claim 31 or claim 32 wherein said vertebrate is avian.

## Patentansprüche

1. Verfahren zur Herstellung einer kontinuierlichen Zelllinie, die fähig ist, das Wachstum eines zellassoziierten alpha-Herpesvirus zu unterstützen, umfassend Infizieren oder Transfizieren einer Zelle mit einer rekombinanten Nucleinsäure eines Herpesvirus und Kultivieren der infizierten oder transfizierten Zelle oder Nachkommenschaft derselben unter Bedingungen, die für die Expression der Nucleinsäure und die Vermehrung der Zelle oder Nachkommenschaft derselben geeignet sind, wobei die Zelle oder die Nachkommenschaft derselben frei von einem Säuger- oder aviären Virus ist und wobei die Nucleinsäure ein Herpesvirus-Glycoprotein gE-Gen oder ein funktionelles Fragment davon umfasst.

2. Verfahren gemäß Anspruch 1, wobei die infizierte oder transfizierte Zelle eine Vertebratenzelle ist.

3. Verfahren gemäß Anspruch 1 oder Anspruch 2, wobei die infizierte oder transfizierte Zelle eine aviäre Zelle bzw. Vogelzelle bzw. Geflügelzelle ist.

4. Verfahren gemäß einem der Ansprüche 1 bis 3, wobei die infizierte oder transfizierte Zelle eine Muskel-Myoblastenzelle ist.

5. Verfahren gemäß Anspruch 4, wobei die Muskel-Myoblastenzelle eine QM7-Zelle ist.

6. Verfahren gemäß Anspruch 5, wobei die QM7-Zelle von einer Zelle abgeleitet ist, die als ATCC CRL-1632 hinterlegt wurde.

7. Verfahren gemäß einem der Ansprüche 1 bis 6, wobei das Herpesvirus ein Virus der Marekschen Krankheit, vorzugsweise Serotyp 1, 2 oder 3, ist.

8. Verfahren gemäß Anspruch 7, wobei das Virus der Marekschen Krankheit ein avirulentes Virus der Marekschen Krankheit ist.

9. Verfahren gemäß Anspruch 8, wobei der avirulente Stamm Rispens CVI988 ist.

10. Verfahren gemäß einem der Ansprüche 1 bis 9, wobei die kontinuierliche Zelllinie fähig ist, das Wachstum eines Virusstamms der Marekschen Krankheit ohne Adaption des Virus zu unterstützen.

11. Zelle oder Nachkommenschaft derselben, frei von einem Säuger- oder aviären Virus, einer kontinuierlichen Zelllinie, die fähig ist, das Wachstum eines zellassoziierten alphaHerpesvirus zu unterstützen, umfassend eine rekombinante Nucleinsäure eines Herpesvirus, wobei die Nucleinsäure ein Herpesvirus-Glycoprotein gE-Gen oder ein funktionelles Fragment davon umfasst.

12. Zellpräparation, erhalten aus einer Zelle oder einer Nachkommenschaft derselben gemäß Anspruch 11.

13. Verwendung einer Zelle gemäß Anspruch 11 und/oder einer Präparation gemäß Anspruch 12 für die Herstellung eines Vakzins, das fähig ist, Schutz gegen eine Krankheit in einem Vertebraten zu induzieren.

14. Verwendung gemäß Anspruch 13, wobei der Vertebrate aviär ist.

15. Verwendung einer Zelle gemäß Anspruch 11 für die Entwicklung und/oder Aufrechterhaltung und/oder Isolierung eines hypervirulenten und/oder sehr virulent-plus und/oder sehr virulenten Stamms und/oder virulenten und/oder avirulenten Virusstamms der Marekschen Krankheit.

16. Verwendung gemäß Anspruch 15, außerdem umfassend Kultivieren der Zelle mit dem Virus in Gegenwart von Geniticin.

17. Verwendung gemäß Anspruch 15 oder Anspruch 16, wobei der Virusstamm der Marekschen Krankheit ein Virus-Serotyp 1 der Marekschen Krankheit ist.

18. Verwendung gemäß einem der Ansprüche 15 bis 17, wobei der Virusstamm der Marekschen Krankheit EU1 oder RB1B oder 584Ap80C oder CVI988 ist.

19. Verwendung gemäß einem der Ansprüche 15 bis 18, wobei der Virusstamm der Marekschen Krankheit ein natürlicher oder genetisch modifizierter Stamm ist.

20. Verwendung einer Zelle gemäß Anspruch 11 für die Herstellung eines diagnostischen Antigens eines Virus der Marekschen Krankheit.

21. Verfahren, um einen Virusstamm der Marekschen Krankheit zu entwickeln und/oder zu isolieren und/oder aufrecht zu erhalten, umfassend Infizieren einer Zelle gemäß Anspruch 11 mit dem Stamm und Kultivieren der Zelle unter Bedingungen, die für die Vermehrung der Zelle und die Entwicklung, Aufrechterhaltung und Isolierung des Virus der Marekschen Krankheit geeignet sind.

22. Verfahren gemäß Anspruch 21, umfassend Kultivieren der Zelle mit dem Virus in Gegenwart von Geniticin.

23. Verfahren gemäß Anspruch 21 oder Anspruch 22, wobei der Virusstamm der Marekschen Krankheit Virus-Serotyp 1, -Serotyp 2 oder -Serotyp 3 der Marekschen Krankheit ist.

24. Verfahren gemäß einem der Ansprüche 21 bis 23, wobei der Virusstamm der Marekschen Krankheit EU1 oder RB1B oder 584Ap80C oder CVI988 ist.

25. Verfahren gemäß einem der Ansprüche 21 bis 24, wobei der Virusstamm der Marekschen Krankheit ein Vakzinstamm ist.

26. Verfahren zur Herstellung eines diagnostischen Antigens eines Virus der Marekschen Krankheit, umfassend Bereitstellen einer Zelle gemäß Anspruch 11 und Isolieren des Antigens daraus.

27. Verfahren zur Herstellung eines Vakzins, das fähig ist, Schutz gegen eine Krankheit in einem Vertebraten zu induzieren, umfassend Kultivieren einer Zelle gemäß Anspruch 11 und Ernten von Zellen oder Virus-enthaltenden Komponenten daraus.

28. Verfahren gemäß Anspruch 27, wobei der Vertebrate aviär ist.

29. Vakzin, das fähig ist, Schutz gegen eine Krankheit in einem Vertebraten zu induzieren, wobei das Vakzin geerntete Zellkomponenten aus einer kultivierten Zelle gemäß Anspruch 11 umfasst.

30. Vakzin gemäß Anspruch 29, wobei der Vertebrate aviär ist.

31. Verwendung eines Vakzins gemäß Anspruch 29 bei der Herstellung eines Medikaments für die Prävention und/oder Behandlung einer Herpesvirus-induzierten Krankheit.

32. Verwendung gemäß Anspruch 31, wobei die Krankheit eine mit einem Virus der Marekschen Krankheit assoziierte Krankheit ist.

33. Verwendung gemäß Anspruch 31 oder Anspruch 32, wobei der Vertebrate aviär ist.

## Revendications

1. Procédé de production d'une lignée cellulaire continue apte à permettre la croissance d'un herpèsvirus alpha associé à une cellule, comprenant les étapes consistant à infecter ou transfecter une cellule avec un acide nucléique recombinant d'un herpèsvirus et à cultiver ladite cellule infectée ou transfectée ou sa descendance dans des conditions propices à l'expression dudit acide nucléique et à la propagation de ladite cellule ou de la descendance de ladite cellule, dans lequel ladite cellule ou la descendance de ladite cellule est exempte de virus de mammifère et de virus aviaire, et dans lequel ledit acide nucléique contient un gène codant pour la glycoprotéine gE de l'herpèsvirus ou un fragment fonctionnel de ce gène.

2. Procédé selon la revendication 1, dans lequel ladite cellule infectée ou transfectée est une cellule de vertébré.

3. Procédé selon la revendication 1 ou la revendication 2, dans lequel ladite cellule infectée ou transfectée est une cellule aviaire.

4. Procédé selon l'une quelconque des revendications 1 à 3, dans lequel ladite cellule infectée ou transfectée est une cellule musculaire myoblastique.

5. Procédé selon la revendication 4, dans lequel ladite cellule musculaire myoblastique est une cellule QM7.

6. Procédé selon la revendication 5, dans lequel ladite cellule QM7 est dérivée d'une cellule déposée sous la référence ATCC CRL-1632.

7. Procédé selon l'une quelconque des revendications 1 à 6, dans lequel ledit herpèsvirus est un virus de la maladie de Marek, de préférence de sérotype 1, 2 ou 3.

8. Procédé selon la revendication 7, dans lequel ledit virus de la maladie de Marek est un virus avirulent de la maladie de Marek.

9. Procédé selon la revendication 8, dans lequel ladite souche avirulente est une souche Rispens CVI988.

10. Procédé selon l'une quelconque des revendications 1 à 8, dans lequel ladite lignée cellulaire continue est apte à permettre la croissance d'une souche de virus de la maladie de Marek sans adaptation dudit virus.

11. Cellule ou descendance de ladite cellule, exempte de virus de mammifère et de virus aviaire, issue d'une lignée cellulaire continue apte à permettre la croissance d'un herpèsvirus alpha associé à une cellule, contenant un acide nucléique recombinant d'un herpèsvirus, dans laquelle ledit acide nucléique contient un gène codant pour la glycoprotéine gE de l'herpèsvirus ou un fragment fonctionnel de ce gène.

12. Préparation cellulaire obtenue à partir d'une cellule selon la revendication 11 ou de sa descendance.

13. Utilisation d'une cellule selon la revendication 11 et/ou préparation selon la revendication 12 pour la préparation d'un vaccin capable de conférer une protection contre la maladie chez un vertébré.

14. Utilisation selon la revendication 13, dans laquelle ledit vertébré est un oiseau.

15. Utilisation d'une cellule selon la revendication 11 pour la génération et/ou le maintien en culture et/ou l'isolement d'une souche hypervirulente et/ou très très virulente et/ou très virulente et/ou virulente et/ou avirulente de virus de la maladie de Marek.

16. Utilisation selon la revendication 15, comprenant en outre la mise en culture de ladite cellule avec ledit virus en présence de généticine.

17. Utilisation selon la revendication 15 ou la revendication 16, dans laquelle ladite souche de virus de la maladie de Marek est un virus de la maladie de Marek de sérotype 1.

18. Utilisation selon l'une quelconque des revendications 15 à 17, dans laquelle ladite souche de virus de la maladie de Marek est EU1 ou RB1B ou 584Ap80C ou CVI988.

19. Utilisation selon l'une quelconque des revendications 15 à 18, dans laquelle ladite souche de virus de la maladie de Marek est une souche naturelle ou une souche génétiquement modifiée.

20. Utilisation d'une cellule selon la revendication 11 pour la production d'un antigène diagnostique d'un virus de la maladie de Marek.

21. Procédé permettant de générer et/ou isoler et/ou maintenir en culture une souche de virus de la maladie de Marek comprenant les étapes consistant à infecter une cellule selon la revendication 11 avec ladite souche et à cultiver ladite cellule dans des conditions propices à la propagation de ladite cellule et à la génération, au maintien en culture et à l'isolement du virus de la maladie de Marek.

22. Procédé selon la revendication 21, comprenant l'étape consistant à cultiver ladite cellule contenant ledit virus en présence de généticine.

23. Procédé selon la revendication 21 ou la revendication 22, dans lequel ladite souche de virus de la maladie de Marek est un virus de la maladie de Marek de sérotype 1, de sérotype 2 ou de sérotype 3.

24. Procédé selon l'une quelconque des revendications 21 à 23, dans lequel ladite souche de virus de la maladie de Marek est EU1 ou RB IB ou 584Ap80C ou CVI988.

25. Procédé selon l'une quelconque des revendications 21 à 24, dans lequel ladite souche de virus de la maladie de Marek est une souche vaccinale.

26. Procédé de production d'un antigène diagnostique d'un virus de la maladie de Marek, comprenant les étapes consistant à fournir une cellule selon la revendication 11 et à en isoler ledit antigène.

27. Procédé permettant de préparer un vaccin capable de conférer une protection contre la maladie chez un vertébré, comprenant les étapes consistant à cultiver une cellule selon la revendication 11 et à récolter, à partir de cette culture, des cellules ou des composants contenant le virus.

28. Procédé selon la revendication 27, dans lequel ledit vertébré est un oiseau.

29. Vaccin capable de conférer une protection contre la maladie chez un vertébré, ledit vaccin comprenant des composants cellulaires récoltés à partir de la culture d'une cellule selon la revendication 11.

30. Vaccin selon la revendication 29, dans lequel ledit vertébré est un oiseau.

31. Utilisation d'un vaccin selon la revendication 29 dans la préparation d'un médicament destiné à prévenir et/ou à traiter une maladie provoquée par un herpèsvirus.

32. Utilisation selon la revendication 31, dans laquelle ladite maladie est une maladie associée au virus de la maladie de Marek.

33. Utilisation d'un vaccin selon la revendication 31 ou la revendication 32, dans laquelle ledit vertébré est un oiseau.
